# DEMANDE DE BREVET EUROPEEN

(11) **EP 4 501 919 A1**
(43) Date de publication de la demande: **05.02.2025**
(21) Numéro de dépôt: 24192026.3
(22) Date de dépôt: 31.07.2024
(51) Int. Cl.: C07D 307/60

(54) **PROCEDE DE PREPARATION D ANHYDRIDE CYCLIQUE A PARTIR D'UNE LACTONE**

(30) Priorité: 01.08.2023 FR 2308320
(71) Demandeur: Commissariat à l'Energie Atomique et aux Energies Alternatives, 75015 Paris (FR); Centre National de la Recherche Scientifique, 75016 Paris (FR)
(72) Inventeur: PIETRARU, Marie-Hélène, 91191 GIF-SUR-YVETTE CEDEX (FR); NICOLAS, Emmanuel, 91191 GIF-SUR-YVETTE CEDEX (FR); CANTAT, Thibault, 91191 GIF-SUR-YVETTE CEDEX (FR)
(74) Mandataire: Gevers & Orès

(57) **Abrégé**

La présente invention concerne un procédé de préparation d'anhydride cyclique notamment d'anhydride succinique et d'anhydride méthylsuccinique à partir d'une β-lactone, notamment de β-propiolactone et de β-butyrolactone, respectivement, en présence de monoxyde de carbone, catalysé par l'octacarbonyle de dicobalt [Co₂(CO)₈], dans un solvant organique.

L'invention concerne également l'utilisation de ce procédé dans la fabrication d'additifs alimentaires, de plastifiants, de polymères d'intérêt notamment les polyesters, les polyuréthanes et les élasthannes, de résines, de revêtements, de produits pharmaceutiques.

## Description

### Domaine technique de l'invention

La présente invention concerne un procédé de préparation d'anhydride cyclique notamment d'anhydride succinique et d'anhydride méthylsuccinique à partir d'une β-lactone, notamment de β-propiolactone et de β-butyrolactone, respectivement, en présence de monoxyde de carbone, catalysé par l'octacarbonyle de dicobalt [Co₂(CO)₈], dans un solvant organique.

L'invention concerne également l'utilisation de ce procédé dans la fabrication d'additifs alimentaire, de plastifiants, de polymères d'intérêt notamment les polyesters, les polyuréthanes et les élasthannes, de résines, de revêtements, de produits pharmaceutiques.

### Arrière-plan technique

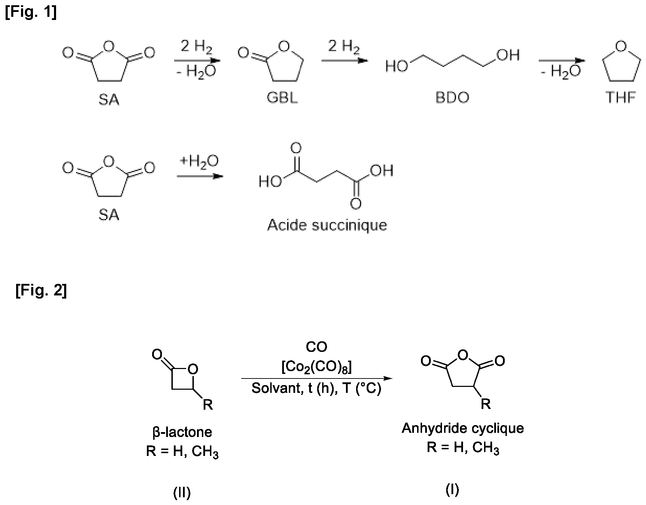

L'anhydride succinique est une molécule d'intérêt industriel. L'anhydride succinique est utilisé comme monomère, par exemple, lors de la synthèse de polyesters aliphatiques via une copolymérisation catalytique avec un époxyde. L'anhydride succinique peut également être un intermédiaire dans la synthèse d'autres molécules d'intérêt : son hydratation donne le diacide correspondant, l'acide succinique, et sa déshydrogénation donne la γ-butyroiactone (GBL) puis le 1,4-butanediol (BDO), qui peut à son tour être déshydraté en tétrahydrofurane (THF). Ces réactions sont schématisées en [Fig. 1].

A la connaissance des inventeurs, seuls deux exemples décrivent un procédé de synthèse d'anhydride succinique par carbonylation de la β-propiolactone en l'absence d'additif acide de Lewis :
- Le procédé décrit par Tsuji en 1969 (Y. Mori, J. Tsuji, Bull. Chem. Soc. Jpn. 1969, 42, 777. doi.org/10.1246/bcsj.42.777). En présence de [Co₂(CO)₈] (2,5 mol%) comme catalyseur, sous une pression de monoxyde de carbone de 100 bar, dans le benzène, la β-propiolactone est convertie en anhydride succinique avec un rendement de 29 %, après 4 h à 150 °C. Ainsi, ce procédé donne un rendement modeste en anhydride succinique, et ce dans des conditions contraignantes, notamment pour ce qui est de la pression.
- Le procédé décrit par Novomer (WO2022221086) décrivant une méthode pour carbonyler un époxyde ou une lactone en la faisant réagir avec du CO en présence d'un catalyseur à une température supérieure à 80 °C pour former un produit de carbonylation. Néanmoins, les exemples donnés par les inventeurs de la présente invention montrent que l'activité catalytique est très dépendante du choix de la combinaison de certains paramètres de la réaction, notamment la pression et le solvant : dans certains cas, aucune activité catalytique n'est observée ; alors que dans d'autres, de l'activité est observée.

En dehors de ces deux exemples, la synthèse de l'anhydride succinique par carbonylation de la β-propiolactone n'a été décrite dans la littérature qu'en présence d'un additif acide de Lewis comme co-catalyseur.

Tout type d'acide de Lewis a été protégé ou mentionné dans les publications et demandes de brevet déposées par le groupe de Coates ou les demandes de brevet déposées par sa société Novomer (Coates G. W. J. Am. Chem. Soc. 2004, 126, 6842. doi.org/10.1021/ja048946m ; WO 03/050154 ; US 2007/0213524 ; WO 2012/158573 ; WO 2015/138975). Ce procédé s'est montré efficace pour la carbonylation de lactones en anhydride cyclique. Le catalyseur et l'additif de type d'acide de Lewis, peuvent être introduits séparément, ou bien de manière combinée sous la forme d'un sel ionique. Les catalyseurs les plus efficaces en termes d'activité et/ou de sélectivité sont généralement constitués d'un métal carbonyle anionique [Co(CO)₄]⁻ et d'un acide de Lewis cationique de type [(ligand)M(solvant)₂]⁺, avec par exemple,
M = Al ou Cr ;
solvant = THF,
ligand = salph, oep, tpp (salph : *N,N*'-bis(3,5-di-*tert*-butylsalicylidene)phenyldiamino ;
oep : octaethylporphyrine ; tpp : tétraphénylporphyrine).

Ces acides de Lewis cationiques et les catalyseurs correspondants peuvent demander une à plusieurs étapes de synthèse.

Ce type d'acide de Lewis associé à l'anion [Co(CO)₄]⁻a également été décrit sous la forme de réseaux métallo-organiques par le groupe de Dinc 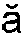 et Román-Leshkov, et ce procédé s'est montré efficace pour la carbonylation de lactones en anhydride cyclique (D.P. Hoyoung, M. Dinc 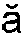 , Y. Román-Leshkov, *J. Am. Chem. Soc.* **2018,** *140*, 10669. doi.org/10.1021/jacs.8b05948).

Il existe donc un réel besoin d'un procédé permettant la synthèse d'un anhydride cyclique de manière durable à partir de réactifs ou solvants qui peuvent être biosourcés et dans des conditions de pression et de température relativement douces pour réduire la consommation énergétique nécessaire à la synthèse de l'anhydride succinique.

En particulier, il existe un réel besoin d'un procédé de synthèse d'un anhydride cyclique qui soit facile à mettre en oeuvre et intéressant sur le plan industriel. Plus particulièrement, il existe un réel besoin d'un procédé de synthèse d'un anhydride cyclique qui ne nécessite pas l'utilisation d'un additif de type acide de Lewis. Tous les réactifs et solvants nécessaires devraient être disponibles commercialement, sans étape de synthèse préalable.

### Résumé de l'invention

La présente invention a précisément pour but de répondre à ces besoins en fournissant un procédé de préparation un procédé de préparation d'un anhydride cyclique de formule (I) 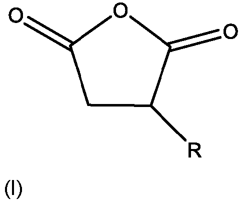 dans laquelle
R représente un atome d'hydrogène ou un radical méthyle, caractérisé en ce que l'on fait réagir une β-lactone de formule (II) 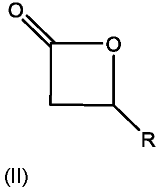
dans laquelle R représente un atome d'hydrogène ou un radical méthyle,
avec du monoxyde de carbone (CO), en présence de l'octacarbonyle de dicobalt de formule [Co₂(CO)₈] comme catalyseur, dans un ou un mélange d'au moins deux solvant(s) choisi(s) parmi :
   - les cétones, en particulier, l'acétone, la methyléthylcétone (MEC), la méthylisobutylcétone (MIBC), la cyclohexanone ;
   - les esters, en particulier, l'acétate d'éthyle, l'acétate de méthyle, l'acétate d'isopropyle, l'acétate de butyle, l'acétate d'isobutyle, l'acétate d'isoamyle, le diacétate de glycol, la γ-valérolactone, le succinate de diéthyle ;
   - les éthers, en particulier, le tétrahydrofurane, le méthyltétrahydrofurane, le 1,4-dioxane, l'anisole, le diméthoxyethane, l'éther diéthylique, l'éther diisopropylique, l'éther méthylique *tert*-butylique, l'éther méthylique *tert*-amylique, l'éther méthylique cyclopentylique, l'éther éthylique *tert*-butylique ;
   - les hydrocarbures aromatiques, en particulier, le toluène, l'éthylbenzène, le m- et p-xylène, le mésitylène, le styrène ;
   - les solvants polaires aprotiques, en particulier, l'acétonitrile, la *N,N*'-diméthylpropylène urée, le diméthylsulfoxyde, le sulfolane, le nitrométhane, le carbonate de diméthyle, le carbonate d'éthylène, le carbonate de propylène ;
à une température inférieure ou égale à 150°C, et à une pression totale en monoxyde de carbone (CO) inférieure ou égale à 60 bar.

Les conditions dans lesquelles le procédé de l'invention est mis en oeuvre sont particulièrement avantageuses :
- les conditions utilisées sont douces ce qui permet de réduire la consommation énergétique nécessaire à la synthèse du produit ;
- une séparation aisée de l'anhydride cyclique de formule (I) des autres espèces du milieu réactionnel est possible notamment par un choix de solvant dans lequel ledit anhydride cyclique a une très faible solubilité.

Le choix approprié du solvant et l'application d'une pression de monoxyde de carbone sont des paramètres importants pour la mise en oeuvre du procédé de l'invention. Par ailleurs, le procédé de l'invention a l'avantage d'éviter l'utilisation d'un additif acide de Lewis.

L'invention concerne également l'utilisation de ce procédé dans la fabrication d'additifs alimentaires, de plastifiants, de polymères d'intérêt notamment les polyesters, les polyuréthanes et les élasthannes, de résines, de revêtements, de produits pharmaceutiques.

### Brève description des figures

D'autres caractéristiques et avantages de l'invention apparaitront au cours de la lecture de la description détaillée qui va suivre pour la compréhension de laquelle on se reportera aux figures annexées dans lesquelles :
[Fig. 1] représente l'utilisation de l'anhydride succinique (SA) comme réactif de départ pour la synthèse d'autres molécules d'intérêt industriel.
[Fig. 2] représente la synthèse d'un anhydride cyclique de formule (I) à partir d'une β-lactone de formule (II), selon le procédé de l'invention.

### Description détaillée de l'invention

La présente invention concerne un procédé de préparation d'un anhydride cyclique de formule (I) 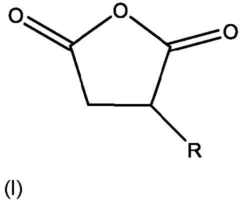 dans laquelle
R représente un atome d'hydrogène ou un radical méthyle, caractérisé en ce que l'on fait réagir une β-lactone de formule (II) 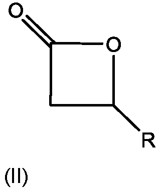
dans laquelle R représente un atome d'hydrogène ou un radical méthyle,
avec du monoxyde de carbone (CO), en présence de l'octacarbonyle de dicobalt de formule [Co₂(CO)₈] comme catalyseur, dans un ou un mélange d'au moins deux solvant(s) choisi(s) parmi :
   - les cétones, en particulier, l'acétone, la methyléthylcétone (MEC), la méthylisobutylcétone (MIBC), la cyclohexanone ;
   - les esters, en particulier, l'acétate d'éthyle, l'acétate de méthyle, l'acétate d'isopropyle, l'acétate de butyle, l'acétate d'isobutyle, l'acétate d'isoamyle, le diacétate de glycol, la γ-valérolactone, le succinate de diéthyle ;
   - les éthers, en particulier, le tétrahydrofurane (THF), le méthyltétrahydrofurane, le 1,4-dioxane, l'anisole, le diméthoxyéthane, l'éther diéthylique, l'éther diisopropylique, l'éther méthylique *tert*-butylique, l'éther méthylique *tert*-amylique, l'éther méthylique cyclopentylique, l'éther éthylique *tert*-butylique ;
   - les hydrocarbures aromatiques, en particulier, le toluène, l'éthylbenzène, le m- et p-xylène, le mésitylène, le styrène ;
   - les solvants polaires aprotiques, en particulier, l'acétonitrile, la *N,N*'-diméthylpropylène urée, le diméthylsulfoxyde, le sulfolane, le nitrométhane, le carbonate de diméthyle, le carbonate d'éthylène, le carbonate de propylène ;
à une température inférieure ou égale à 150°C, et à une pression totale en monoxyde de carbone (CO) inférieure ou égale à 60 bar.

Ainsi, le procédé de l'invention ne nécessite pas l'utilisation d'un additif de type acide de Lewis. Tous les réactifs et solvants nécessaires sont disponibles commercialement, sans étape de synthèse préalable.

D'autres avantages du procédé de l'invention résident dans les conditions douces utilisées permettent de réduire la consommation énergétique nécessaire à la synthèse du produit.

Par ailleurs, des solvants « recommandés » quant à leur sécurité, toxicité, et impact environnemental peuvent être utilisés dans le procédé de l'invention (Prat, D., Wells, A., Hayler, J., Sneddon, H., McElroy, C. R., Abou-Shehada, S., & Dunn, P. J. Green Chemistry, 2016, 18, 288 ; doi.org/10.1039/C5GC01008J).

Les variantes du procédé de l'invention consistent dans le choix du solvant, la pression en monoxyde de carbone, la température de réaction, le temps de réaction, la concentration initiale en lactone et la charge catalytique.

Dans un mode de réalisation préféré de l'invention, le ou le mélange d'au moins deux solvant(s) est choisi(s) parmi :
- l'acétone ;
- l'acétate d'éthyle ;
- le 1,4-dioxane ;
- le tétrahydrofurane (THF) ;
- l'anisole ;
- le diméthoxyéthane ;
- l'acétonitrile ;
- le carbonate de diméthyle ;
- le carbonate d'éthylène ;
- le carbonate de propylène ; et
- le toluène.

Dans un autre mode de réalisation préféré de l'invention, le ou le mélange d'au moins deux solvant(s) est choisi(s) parmi :
- l'acétone ;
- l'acétate d'éthyle ;
- le 1,4-dioxane ;
- le tétrahydrofurane (THF) ;
- l'anisole ;
- le diméthoxyéthane ;
- l'acétonitrile ;
- le carbonate de diméthyle ;
- le toluène.

Dans un mode autre de réalisation préféré de l'invention, le solvant est l'acétonitrile. La pression totale en monoxyde de carbone peut varier de 1 bar à 60 bar selon le solvant, la charge catalytique, la température de la réaction, la concentration en lactone, et le temps de réaction désiré.

Dans un mode de réalisation de l'invention, le procédé de l'invention se produit sous une pression totale en monoxyde de carbone (CO) de 1 à 60 bar.

Dans un mode de réalisation préféré de l'invention, le procédé de l'invention se produit sous une pression totale en monoxyde de carbone (CO) de 5 à 50 bar. Dans un autre mode préféré de l'invention, la pression totale en monoxyde de carbone (CO) est de 5 à 20 bar.

La température de réaction peut varier de 30 °C à 150 °C selon le solvant, la charge catalytique, la concentration en lactone et le temps de réaction désiré.

Dans un mode de réalisation préféré de l'invention, la température est entre 60 et 150°C. Dans un autre mode préféré de l'invention, la température est entre 60 et 130°C. Selon un autre mode de réalisation préféré de l'invention, la température est entre 90 et 130°C. Selon un autre mode de réalisation préféré de l'invention, la température est entre 90 et 110°C.

Dans le procédé de l'invention, la quantité de l'octacarbonyle de dicobalt de formule [Co₂(CO)₈], en tant que catalyseur, est de 0,1 à 10 mol%, de préférence de 0,5 à 8 mol%, plus préférentiellement de 1 à 6 mol%, par rapport à la quantité de la lactone de formule (II). L'augmentation de la quantité du catalyseur permet de diminuer le temps de réaction.

La concentration en lactone de formule (II) telle que défini ci-dessus, peut varier de plusieurs mmol.L⁻¹ à plusieurs mol.L⁻¹. Selon un mode de réalisation de l'invention, la concentration en lactone de formule (II) est de 0,01 à 6 M. Selon un mode de réalisation préféré de l'invention, la concentration en lactone de formule (II) est comprise entre 0,1 et 2 M. Selon un mode de réalisation de l'invention encore plus préféré, la concentration en lactone de formule (II) est comprise entre 0,25 et 1 M. Le temps de réaction peut varier de quelques minutes à quelques jours selon la lactone, le solvant, la charge catalytique, la concentration en lactone et la température. La durée du procédé de l'invention est comprise entre 30 minutes et 150 heures. De préférence la durée est entre 6 et 110 heures.

Dans un mode de réalisation de l'invention, R représente un atome d'hydrogène.

Dans un autre mode de réalisation de l'invention, R représente un radical méthyle. Lorsque R représente un atome d'hydrogène, l'anhydride cyclique de formule (I) est l'anhydride succinique et la lactone de formule (II) est la β-propiolactone.

Lorsque R représente un radical méthyle, l'anhydride cyclique de formule (I) est l'anhydride méthyle succinique et la lactone de formule (II) est la β-butyrolactone. Par ailleurs, le procédé de l'invention a pour vocation de s'appliquer facilement au niveau industriel et être compétitif par rapport aux procédés déjà mis en oeuvre à l'échelle industrielle, puisqu'il met en l'octacarbonyle de dicobalt [Co₂(CO)₈], déjà éprouvés dans des procédés industriels d'hydroformylation, qui peuvent être utilisés ici dans des conditions de pression et de température relativement douces (telles que par exemple, 60 bar ou moins et 150°C ou moins).

Contrairement aux procédés connus, le procédé de l'invention ne nécessite pas l'ajout d'un additif supplémentaire pour améliorer les performances du procédé. Dans le cadre de l'invention, « additif » désigne un composé qui est un acide de Lewis, apte à améliorer la réactivité des réactifs pour la transformation de la lactone de formule (II) en anhydride cyclique de formule (I).

Par ailleurs, un autre avantage du procédé de l'invention est la simplicité de sa mise en oeuvre : outre l'absence d'additif acide de Lewis, l'octacarbonyle de dicobalt [Co₂(CO)₈] et le(s) solvant(s) sont de produits commerciaux et ne nécessitent donc pas d'étape supplémentaire de synthèse. Le procédé est efficace à basse pression, en particulier, inférieure et égale à 60 bar, ce qui est particulièrement avantageux d'un point de vue industriel.

L'invention concerne également l'utilisation de ce procédé dans la fabrication d'additifs alimentaires, de plastifiants, de polymères d'intérêt notamment les polyesters, les polyuréthanes et les élasthannes, de résines, de revêtements, de produits pharmaceutiques.

### Description expérimentale de l'invention :

Les différents réactifs, catalyseurs, additifs et solvants utilisés directement ou indirectement dans le procédé de l'invention notamment, sont, en général, des composés/solvants commerciaux.

Un autre objet de l'invention est l'utilisation d'un procédé selon l'invention, dans la fabrication d'additifs alimentaires, de plastifiants, de polymères d'intérêt notamment les polyesters, les polyuréthanes et les élasthannes, de résines, de revêtements, de produits pharmaceutiques.

Le procédé de fabrication d'additifs alimentaires, de plastifiants, de polymères d'intérêt notamment les polyesters, les polyuréthanes et les élasthannes, de résines, de revêtements, de produits pharmaceutiques, peut comprendre une étape de préparation d'un anhydride cyclique de formule (I), notamment d'anhydride succinique, par le procédé de l'invention.

### EXEMPLES

Les différents réactifs et solvants utilisés dans le procédé de l'invention et dans les exemples sont, en général, des composés commerciaux ou peuvent être préparés par tout procédé connu de l'homme du métier.

Sauf précisé, toutes les réactions ont été menées sous argon, en atmosphère anhydre et inerte (< 5 ppm de H₂O et de O₂) en utilisant les techniques classiques en schlenk avec une rampe sous vide ou dans une boîte à gants (mBraun LabMaster DP). La verrerie a été séchée plusieurs heures à 120°C dans une étuve avant utilisation. Les solvants de réaction ont été préalablement séchés par des méthodes usuelles, distillés et conservés en atmosphère inerte sur du tamis moléculaire 4 Å. Le tamis moléculaire 4 Å (Aldrich) a été préalablement séché sous vide dynamique à 250°C pendant 48 h avant utilisation. Tous les produits ont été achetés chez les fournisseurs classiques (Sigma Aldrich, Strem, Alfa-Aesar, Acros, etc), et préalablement séchés ou dégazés si nécessaire. Le monoxyde de carbone provient de bouteilles sous pression de pureté 4.7 de Air Products.

Les composés synthétisés ont été caractérisés et quantifiés par les techniques d'analyse et de spectrométrie de masse grâce à l'instrument Shimadzu GCMS QP2010 Ultra, équipé d'une colonne en silice Supelco SLB-ms (30 m x 0,25 mm x 0,25 µm). Le gaz porteur est de l'hélium (pureté 6.0) de chez Messer. Les produits ont été identifiés par comparaison avec les standards commerciaux, et calibrés en utilisant du mésitylène comme étalon interne.

### Protocole expérimental :

1. Sous atmosphère inerte, l'octacarbonyle de dicobalt [Co₂(CO)₈] est dissous dans le solvant. Selon le solvant utilisé, un dégagement gazeux peut être observé. La lactone est ensuite ajoutée. Afin de suivre le rendement d'anhydride cyclique et la conversion de lactone, un étalon interne comme le mésitylène peut être ajouté.
2. Le mélange réactionnel est introduit dans un autoclave préalablement purgé à trois reprises par un flux de monoxyde de carbone. L'autoclave contient un tube de verre pour recueillir le mélange réactionnel et est muni d'un agitateur magnétique.
3. L'autoclave est ensuite scellé, et le monoxyde de carbone est injecté dans l'autoclave jusqu'à la pression totale désirée.
4. L'autoclave est placé dans un bloc chauffant sous agitation magnétique.
5. La réaction est arrêtée en trempant l'autoclave dans un bain de glace.
6. Une fois que l'autoclave est à température ambiante (20 ± 5°C), il peut être ouvert, et le mélange réactionnel peut être analysé.

Les conversions et rendements décrits sont mesurés en GC-MS (Gas Chromatography - Mass Spectrum) grâce à un étalon interne ajouté en début de réaction, le mésitylène, pour lequel une courbe de calibration a été préalablement réalisée.

### Exemple 1 :

L'octacarbonyle de dicobalt (17,1 mg ; 0,05 mmol ; 0,05 eq) est dissous dans l'acétonitrile (2 mL). Un dégagement gazeux est observé et dure environ 20 min. Le mésitylène (14 µL ; 0,1 mmol ; 0,1 eq) et la β-propiolactone (63 µL ; 1 mmol ; 1 eq) sont ensuite ajoutés au mélange réactionnel. Le mélange réactionnel est introduit dans l'autoclave purgé trois fois par un flux de monoxyde de carbone. L'autoclave est scellé et soumis à une pression de 15 bar de monoxyde de carbone, puis mis sous agitation magnétique pendant 6 h dans un bloc chauffant à 90 °C. Après analyse d'un échantillon de brut réactionnel par GC-MS, 100 % de la β-propiolactone a été convertie, pour un rendement en anhydride succinique de 99 %.

### Exemple 2 :

L'octacarbonyle de dicobalt (17,1 mg ; 0,05 mmol ; 0,05 eq) est dissous dans l'acétonitrile (2 mL). Un dégagement gazeux est observé et dure environ 20 min. Le mésitylène (14 µL ; 0,1 mmol ; 0,1 eq) et la β-butyrolactone (86,1 mg ; 1 mmol ; 1 eq) sont ensuite ajoutés au mélange réactionnel. Le mélange réactionnel est introduit dans l'autoclave purgé trois fois par un flux de monoxyde de carbone. L'autoclave est scellé et soumis à une pression de 15 bar de monoxyde de carbone, puis mis sous agitation magnétique pendant 39 h dans un bloc chauffant à 110 °C. Après analyse d'un échantillon de brut réactionnel par GC-MS, 87 % de la β-butyrolactone a été convertie, pour un rendement en anhydride méthylsuccinique de 67 %.

### Tests réalisés :

Le rendement en anhydride cyclique et la conversion de la lactone ont été obtenus par GC-MS, suite à une calibration réalisée avec des échantillons commerciaux d'anhydride cyclique et de lactone. Les valeurs en pourcentages sont données avec une incertitude de +/- 5 points de pourcentage.

Pour la carbonylation de la β-propiolactone en anhydride succinique (Tableau 1), on remarque que :
- Dans l'acétone, le 1,4-dioxane, le toluène, l'anisole, le carbonate de diméthyle, et l'acétate d'éthyle, la baisse de la pression induit une augmentation du rendement (Tableau 1, entrées 1-7 et 24-35). En particulier, l'augmentation de la pression à 50 bar induit une perte d'activité dans le 1,4-dioxane, l'anisole et le carbonate de diméthyle (Tableau 1, entrées 7, 29 et 32), et cette perte d'activité est observée dès 15 bar dans le toluène (Tableau 1, entrées 25 et 26).
- Dans le diméthoxyéthane, la pression n'a pas d'influence sur le rendement (Tableau 1, entrées 8-10).
- Le DMF est un solvant peu approprié pour le procédé, quelle que soit la pression testée (Tableau 1, entrées 11-13).
- Dans l'acétonitrile et le THF, l'augmentation de la pression de 5 à 15 bar induit l'augmentation du rendement, mais l'augmentation de 15 à 50 bar induit une baisse du rendement (Tableau 1, entrées 14-17 et 21-23). Pour ces deux solvants, une pression modérée est donc préférable.
- Les conditions optimales pour le procédé consistent dans l'utilisation de l'acétonitrile comme solvant, à une pression de 15 bar, où un rendement quantitatif en anhydride est observé après 6 h (Tableau 1, entrée 16).

Les résultats des différentes expériences ont été rassemblés dans les tableaux suivants.

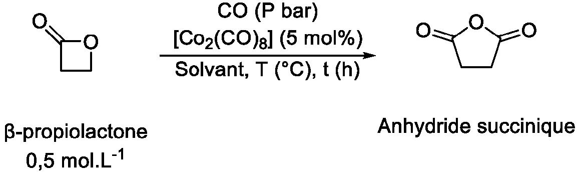

**[Tableau 1]**

| **Tableau 1 - Carbonylation de la β-propiolactone en anhydride succinique.** | | | | | |
|---|---|---|---|---|---|
| **Entrée** | **Solvant** | **T (°C)** | **P (bar)** | **t (h)** | **Rendement en anhydride succinique (%)** |
| 1 | Acétone | 90 | 5 | 6 | 77 |
| 2 | Acétone | 90 | 15 | 6 | 57 |
| 3 | Acétone | 90 | 15 | 15 | 79 |
| 4 | Acétone | 90 | 50 | 6 | 29 |
| 5 | 1,4-dioxane | 90 | 5 | 6 | 76 |
| 6 | 1,4-dioxane | 90 | 15 | 6 | 38 |
| 7 | 1,4-dioxane | 90 | 50 | 6 | < 5 |
| 8 | Diméthoxyéthane | 90 | 5 | 6 | 74 |
| 9 | Diméthoxyéthane | 90 | 15 | 6 | 76 |
| 10 | Diméthoxyéthane | 90 | 50 | 6 | 71 |
| 11 | *N,N*-diméthylformamide | 90 | 5 | 6 | < 5 |
| 12 | *N,N*-diméthylformamide | 90 | 15 | 6 | < 5 |
| 13 | *N,N*-diméthylformamide | 90 | 50 | 6 | < 5 |
| 14 | Acétonitrile | 90 | 5 | 6 | 73 |
| 15 | Acétonitrile | 90 | 15 | 3 | 92 |
| 16 | Acétonitrile | 90 | 15 | 6 | 99 |
| 17 | Acétonitrile | 90 | 50 | 6 | 38 |
| 18 | Acétonitrile | 110 | 15 | 1 | 83 |
| 19 | Acétonitrile | 110 | 15 | 3 | 87 |
| 20 | Acétonitrile | 130 | 15 | 1 | 65 |
| 21 | Tétrahydrofurane | 90 | 5 | 6 | 69 |
| 22 | Tétrahydrofurane | 90 | 15 | 6 | 83 |
| 23 | Tétrahydrofurane | 90 | 50 | 6 | 58 |
| 24 | Toluène | 90 | 5 | 6 | 44 |
| 25 | Toluène | 90 | 15 | 6 | < 5 |
| 26 | Toluène | 90 | 50 | 6 | < 5 |
| 27 | Anisole | 90 | 5 | 6 | 38 |
| 28 | Anisole | 90 | 15 | 6 | 13 |
| 29 | Anisole | 90 | 50 | 6 | < 5 |
| 30 | Carbonate de Diméthyle | 90 | 5 | 6 | 51 |
| 31 | Carbonate de Diméthyle | 90 | 15 | 6 | 13 |
| 32 | Carbonate de Diméthyle | 90 | 50 | 6 | < 5 |
| 33 | Acétate d'éthyle | 90 | 5 | 6 | 76 |
| 34 | Acétate d'éthyle | 90 | 15 | 6 | 37 |
| 35 | Acétate d'éthyle | 90 | 50 | 6 | 16 |
| 36^{a} | Acétonitrile | 90 | 15 | 18 | 92 |
| 37^{b} | Acétonitrile | 90 | 15 | 72 | 60 |
| ^{a} : 2,5 mol% de [Co₂(CO)₈] sont utilisés. | | | | | |
| ^{b} : 1 mol% de [Co₂(CO)₈] est utilisé. | | | | | |

Pour la carbonylation de la β-butyrolactone en anhydride méthylsuccinique (Tableau 2), on remarque que :
- à 90 °C, peu d'activité est observée (Tableau 2, entrée 1).
- l'augmentation de la température à 110 ou 130 °C permet d'observer la formation de l'anhydride, mais une température de 110 °C est préférable pour un meilleur rendement (Tableau 2, entrées 2-4).
- l'augmentation de la pression à 50 bar provoque une diminution du rendement (Tableau 2, entrées 5 et 6).

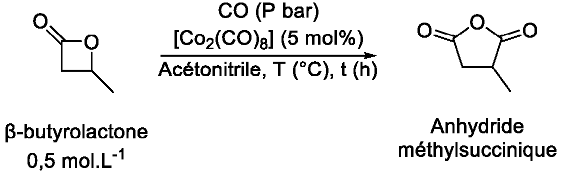

**[Tableau 2]**

| **Tableau 2** - **Carbonylation de la β-butyrolactone en anhydride méthylsuccinique.** | | | | |
|---|---|---|---|---|
| **Entrée** | **P (bar)** | **T(°C)** | **t (h)** | **Rendement en anhydride méthylsuccinique (%)** |
| 1 | 15 | 90 | 6 | < 5 |
| 2 | 15 | 110 | 16 | 48 |
| 3 | 15 | 110 | 39 | 67 |
| 4 | 15 | 130 | 39 | 38 |
| 5 | 50 | 110 | 16 | 31 |
| 6 | 50 | 110 | 72 | 34 |

Les tests que les inventeurs ont réalisés au laboratoire montrent que la combinaison de certains paramètres ne permet pas d'obtenir plus de 5 % de rendement en anhydride succinique ou en anhydride méthylsuccinique. Autrement dit, la combinaison de certains paramètres ne permet d'observer qu'une activité catalytique très faible ou nulle (Tableau 1, **Erreur ! Source du renvoi introuvable**.au 2). Plus précisément, les inventeurs ont constaté une très faible activité catalytique dans les cas suivants, en utilisant [Co₂(CO)₈] comme catalyseur pour la carbonylation de la β-propiolactone, après un chauffage à 90 °C pendant 6 h (Tableau 1) :
- dans le 1,4-dioxane, à 50 bar (Tableau 1, entrée 7) ;
- dans le carbonate de diméthyle, à 50 bar (Tableau 1, entrée 32) ;
- dans le *N,N-*diméthylformamide, à 5, 15 et 50 bar (Tableau 1, entrées 11 à 13) ;
- dans le toluène, à 15 et 50 bar (Tableau 1, entrées 24 et 25).

De même, pour la carbonylation de la β-butyrolactone, catalysée par [Co₂(CO)₈] dans l'acétonitrile à 15 bar, une très faible activité a été observée après 6 h à 90 °C (Tableau 2, entrée 1).

A l'inverse, les inventeurs ont montré qu'une combinaison judicieuse de paramètres permet d'observer une activité catalytique élevée. Ceci est illustré par les autres tests catalytiques décrits pour la carbonylation de la β-propiolactone dans le Tableau 1 :
- l'acétone et l'acétate d'éthyle permettent d'obtenir de bons rendements en anhydride succinique à toute pression, mais en particulier à basse pression (77 % et 76 % à 5 bar, Tableau 1, entrées 1 et 33) ;
- le 1,4-dioxane, l'anisole et le carbonate de diméthyle permettent d'obtenir un rendement modéré ou bon, à pression basse ou modérée (13 à 76 %, Tableau 1, entrées 5, 6, 27, 28, 30 et 31) ;
- le diméthoxyéthane permet d'obtenir un bon rendement à toute pression (71 à 76 %, Tableau 1, entrées 8 à 10) ;
- l'acétonitrile permet d'obtenir un rendement modéré à très bon à toute pression (38 à 99 %, Tableau 1, entrées 14 à 20) ; en particulier, un rendement quantitatif en anhydride succinique est observé sous 15 bar de monoxyde de carbone, après 6 h de réaction à 90 °C (Tableau 1, entrée 16) ;
- le tétrahydrofurane permet d'obtenir un rendement modéré à très bon à toute pression (58 à 83 %, Tableau 1, entrées 21 à 23) ; le rendement le plus élevé est observé à 15 bar (83 %, Tableau 1, entrée 22) ;
- le toluène permet d'obtenir un rendement modéré à basse pression (44 %, Tableau 1, entrée 24).

Ces résultats catalytiques illustrent que les combinaisons des deux paramètres « solvant » et « pression » ne sont pas évidentes, puisqu'aucune tendance claire et systématique ne se dégage. Certains solvants ne sont efficaces à aucune pression (*N,N*-diméthylformamide), d'autres sont efficaces à toute pression (diméthoxyéthane), d'autres sont d'autant plus efficaces que la pression est basse (acétone, 1,4-dioxane, toluène, anisole, carbonate de diméthyle, acétate d'éthyle), et d'autres sont plus efficaces à pression modérée (acétonitrile, tétrahydrofurane).

## Revendications

1. Procédé de préparation d'un anhydride cyclique de formule (I) 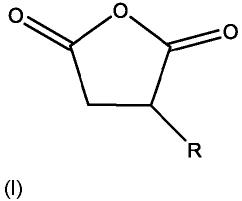 dans laquelle
R représente un atome d'hydrogène ou un radical méthyle, **caractérisé en ce que** l'on fait réagir une β-lactone de formule (II) 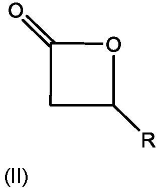
dans laquelle R représente un atome d'hydrogène ou un radical méthyle,
avec du monoxyde de carbone (CO), en présence de l'octacarbonyle de dicobalt de formule [Co₂(CO)₈] comme catalyseur, dans un ou un mélange d'au moins deux solvant(s) choisi(s) parmi :
- les cétones, en particulier, l'acétone, la methyléthylcétone (MEC), la méthylisobutylcétone (MIBC), la cyclohexanone ;
- les esters, en particulier, l'acétate d'éthyle, l'acétate de méthyle, l'acétate d'isopropyle, l'acétate de butyle, l'acétate d'isobutyle, l'acétate d'isoamyle, le diacétate de glycol, la γ-valérolactone, le succinate de diéthyle ;
- les éthers, en particulier, le tétrahydrofurane (THF), le méthyltétrahydrofurane, le 1,4-dioxane, l'anisole, le diméthoxyéthane, l'éther diéthylique, l'éther diisopropylique, l'éther méthylique *tert*-butylique, l'éther méthylique *tert*-amylique, l'éther méthylique cyclopentylique, l'éther éthylique *tert*-butylique ;
- les hydrocarbures aromatiques, en particulier, le toluène, l'éthylbenzène, le m- et p-xylène, le mésitylène, le styrène ;
- les solvants polaires aprotiques, en particulier, l'acétonitrile, le, la *N,N*'-diméthylpropylène urée, le diméthylsulfoxyde, le sulfolane, le nitrométhane, le carbonate de diméthyle, le carbonate d'éthylène, le carbonate de propylène ;
à une température 30 à 150°C, et à une pression totale en monoxyde de carbone (CO) 1 à 60 bar.

2. Procédé selon la revendication 1, **caractérisé en ce que** le ou le mélange d'au moins deux solvant(s) est choisi(s) parmi :
- l'acétone ;
- l'acétate d'éthyle ;
- le 1,4-dioxane ;
- le tétrahydrofurane (THF) ;
- l'anisole ;
- le diméthoxyéthane ;
- l'acétonitrile ;
- le carbonate de diméthyle ; et
- le toluène.

3. Procédé selon l'une des revendications 1 ou 2, **caractérisé en ce que** le solvant est l'acétonitrile.

4. Procédé selon l'une quelconque des revendications 1 à 3, **caractérisé en ce qu'**il se produit sous une pression totale en monoxyde de carbone (CO) de 3 à 50 bar.

5. Procédé selon l'une quelconque des revendications 1 à 4, **caractérisé en ce qu'**il se produit à une température comprise entre 60 et 150°C.

6. Procédé selon l'une quelconque des revendications 1 à 5, **caractérisé en ce que** la quantité de l'octacarbonyle de dicobalt de formule [Co₂(CO)₈], est de 0,1 à 10 mol%, par rapport à la quantité de lactone de formule (II).

7. Procédé selon l'une quelconque des revendications 1 à 6, **caractérisé en ce que** la concentration de lactone de formule (II) est de 0,01 à 6 M.

8. Procédé selon l'une quelconque des revendications 1 à 7, **caractérisé en ce que** la durée du procédé est comprise entre 30 minutes et 150 heures.

9. Procédé selon l'une quelconque des revendications 1 à 8, **caractérisé en ce que** R représentent un atome d'hydrogène.

10. Procédé selon l'une quelconque des revendications 1 à 8, **caractérisé en ce que** R représentent un radical méthyle.

11. Utilisation d'un procédé selon l'une quelconque des revendications 1 à 10, dans la fabrication d'additifs alimentaires, de plastifiants, de polymères d'intérêt notamment les polyesters, les polyuréthanes et les élasthannes, de résines, de revêtements, de produits pharmaceutiques.
